**Europäisches Patentamt**

(19) **European Patent Office**

**Office européen des brevets**

(11) Publication number: **0 248 538**
**A1**

(12) # EUROPEAN PATENT APPLICATION

(21) Application number: **87304039.8**

(22) Date of filing: **06.05.87**

(51) Int. Cl.³: **A 61 M 5/14**

(30) Priority: **06.05.86 US 860207**

(43) Date of publication of application:
**09.12.87 Bulletin 87/50**

(84) Designated Contracting States:
**DE FR GB IT NL**

(71) Applicant: **Colvin, David P.**
**Route nr. 5 Box 67**
**Apex North Carolina 27502(US)**

(72) Inventor: **Colvin, David P.**
**Route nr, 5 Box 67**
**Apex North Carolina 27502(US)**

(74) Representative: **Warren, Anthony Robert et al,**
**BARON & WARREN 18 South End Kensington**
**London W8 5BU(GB)**

(54) Improved infusion method and means.

(57) The invention relates to an infusion pump (10) comprising a frame (11-15) having guide slots (17) for slidably guiding a take-up drum (20). Coil type constant tension springs (26) are each anchored at one end to the bottom (11) of the frame means (11-15) and unsecuringly wound about the tape-up drum (20) at its opposite end. The drum (20) is retracted relative to the anchored ends of the springs (26) to cause the springs (26) to be unwound from the drum (20), and an infusion bag (34) containing a fluid is disposed on the unwound springs (26) with one end engaged in the nip between the drum (20) and unwound springs (26). When the drum (20) is released, the tension exerted by the springs (26) tends to wind up the drum (20), and therefore the infusion bag (34), whereby a substantially constant pressure is applied to the fluid in the bag (34). An infusion tube (35) communicates with the infusion bag (34), and a fixed orifice, flow regulating device including a needle (37) is operatively associated with the infusion tube (35) whereby a constant pressure, predetermined flow rate of fluid from said infusion bag (34) can be assured.

./...

FIG. 5

IMPROVED INFUSION METHOD AND MEANS

This invention relates to medical appliances and more particularly to an improved means and method of infusing liquids into a patient.


## BACKGROUND OF INVENTION


Since the early development of methods of infusing liquids into patients, a problem of flow regulation has been encountered. The standard method that has been almost universally used is the elevated or hanging intravenous fluid bag or bottle. This method works fine in hospital environments where the patient is not being moved, but the same becomes very cumbersome when transport becomes necessary. This problem is even more acute in emergency situations where extra medical personnel are usually in short supply such as at accident scenes and even in ambulance and helicopter transport situations where vertical clearances may be inadequate.

To overcome the above mentioned problems, experimentation has been conducted utilizing mechanical pumps with constant tension springs to drive the same. Two of the most closely related devices of this type are shown in Patent No. 3,670,926 to Hill and Patent No. 3,647,117 to Hargest. In both of these patents, a variable clamp was used in an attempt to control the flow rate. Experimentation has shown, however, that in reality a fixed and known flow rate cannot be accomplished using devices

of this type.    Also, the methods of loading and unloading the infusion bag and attaching the devices in relationship to the patient were not adequate to make the same practical to use.

## BRIEF DESCRIPTION OF INVENTION

After much research and study into the above-mentioned problems, the present invention has been developed to provide a mechanical, gravity independent infusion means with a constant predetermined flow rate.

The above may be accomplished through the use of a plurality of constant force springs which drive a take up drum to apply constant pressure to the infusion bag. The flow from such bag is accurately controlled by a flow regulating needle having a known orifice size. The flow rate can be changed by substitution of alternative sizes of flow regulating needles.

Additionally, the infusion means is easily cocked, loaded, and when infusion has been completed, unloaded. Further, a means for automatically injecting an anticoagulate is provided to prevent loss of vein communication due to a delay in replacing an empty infusion bag which otherwise would lead to blood coagulation and blockage of the infusion needle.

In view of the above, it is an object of the present invention to provide an improved infusion means and method which is gravity independent in operation.

Another object of the present invention is to provide a relatively small, compact infusion means which is sturdy, light weight, and readily storable when not in use.

Another object of the present invention is to provide an improved infusion means into which infusion bags can be readily inserted and removed.

Another object of the present invention is to provide a mechanical infusion means in combination with a visual alarm system.

Another object of the present invention is to provide an infusion means which automatically injects a small quantity of anticoagulate into the system at a predetermined point to prevent loss of vein communication.

Another object of the present invention is to provide a means for automatically regulating pressure in an improved infusion means to prevent further infusion if the vein is lost.

Another object of the present invention is to provide a means for assuring a predetermined constant flow rate from an infusion means.

Another object of the present invention is to provide a plurality of alternative sizes of needles for regulating the flow of fluid from a constant pressure infusion means.

Other objects and advantages of the present invention will become apparent and obvious from a study of the following description and the accompanying drawings which are merely illustrative of such invention.

## BRIEF DESCRIPTION OF DRAWINGS

Figure 1 is a perspective view of the improved infusion means of the present invention in an operative position relative to a patient;

Figure 2 is a bottom perspective view of the present invention showing the attaching clamps associated therewith;

Figure 3 is a top plan view of the improved infusion means of the present invention in cocked position;

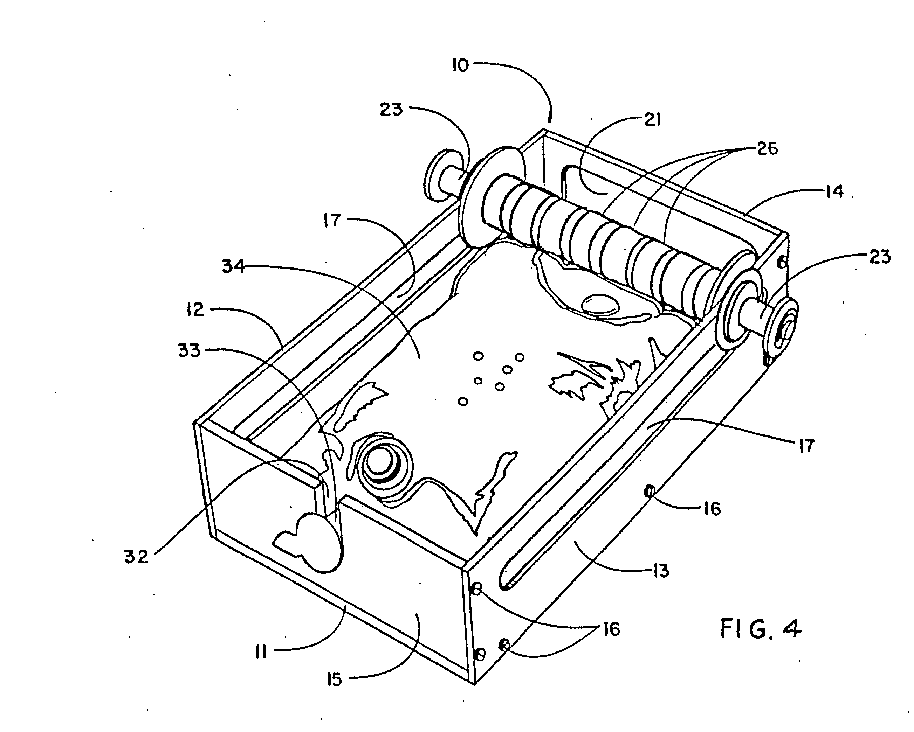

Figure 4 is top plan view of the improved infusion means of the present invention with the infusion bag disposed therein; and

Figure 5 is a top plan view of the improved infusion means of the present invention in actual operation.

## DETAILED DESCRIPTION OF INVENTION

With further reference to the drawings, the improved infusion means of the present invention, indicated generally at 10, is in the form of an open topped tray having a bottom 11 with sides 12 and 13 and ends 14 and 15 upwardly projecting from the periphery thereof. Means for securing the sides, ends and bottom can be by any securing means such as screws 16. Also, weldment could be used as well as fusion and similar methods. Such means for connecting sides, ends and bottoms of various types of materials is well known to those skilled in the art and further detailed discussion of the this portion of the present invention is not deemed necessary.                    0248538

Longitudinal guide slots 17 are provided in sides 12 and 13. At one end of each of these guide slots is an angulated offset slot 18 for locking the draw bar during loading of the infusion bag as will hereinafter be described.

The draw bar 19 passes axially through and mounts take up drum 20. Guide washers 21 are provided on either end of drum 20 adjacent the interior of slides 12 and 13. Adjacent the exterior of sides 12 and 13 are guide washers 22. Cocking handles 23 are provided exteriorly of each of the washers 22 and are held in place by exterior washers 24 and draw bar engaging clips 25. Since clip-type securing means of this type are well known to those skilled in the art, further detailed discussion of the same is not deemed necessary.

A plurality of constant tension coil springs 26 are secured at one end to bottom 11 by means such as spring locking plate 27 and wound about but not otherwise secured to take up draw 20 at the other end. This plate is secured to bottom 11 by means such as screws 28 and to the coil springs 26 by means such as pins 29. Since screw and pin fasteners are well known to those skilled in the art, further detailed discussion of this portion of the present invention is not deemed necessary.

An opening 30 is provided in bottom 11 which can be used for mountingly securing the means of the present invention if desired.

A hand grip opening 31 is provided in end 14 while a relatively deep U-shaped opening 32 with a bulbous lower portion is provided in end 15 for accepting the neck 33. The outer end 0248538

of neck portion 33 of infusion bag 34 is adapted to receive infusion tube 35. A standard pressure clamp 36 is provided on this tube and operates in the normal manner of such devices.

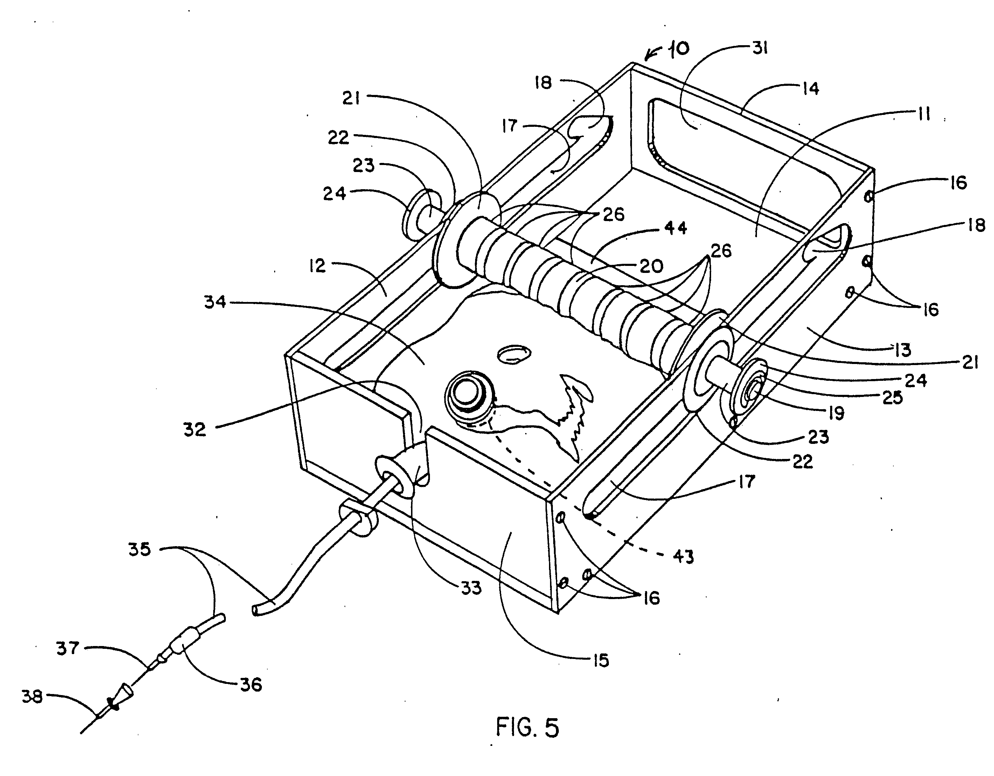

A changeable, flow regulating needle 37 is provided in the end of tube 35 opposite neck 33. A standard infusion needle 38 is shown in exploded fashion in Figure 5 and is adapted to be mounted over flow regulating needle 37 when in use. Since infusion needles and their manner of mounting are well known to those skilled in the art, further detailed discussion of the same is not deemed necessary.

On the exterior of bottom 11 is provided a pair of clips formed from spring steel or similar material. These clips are secured to said bottom by means such as screws or rivits 40.

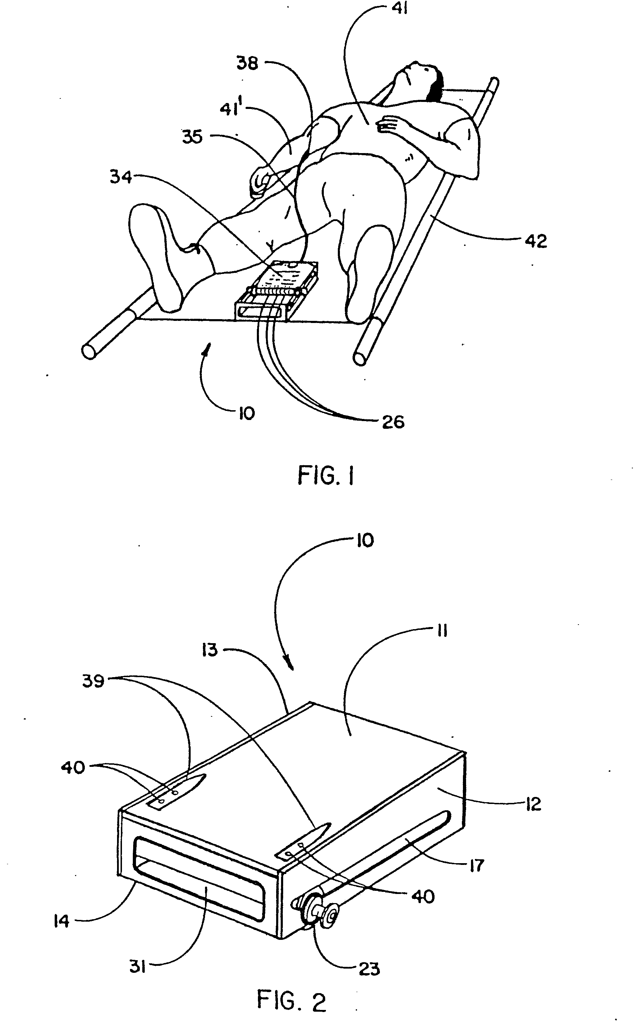

These clips are specifically designed to allow the infusion means 10 of the present invention to be secured to a litter such as that shown in Figure 1.

To use the improved infusion means of the present invention, with the patient 41 lying on a litter or similar means 42, such infusion means is clipped thereonto as shown in Figure 1.

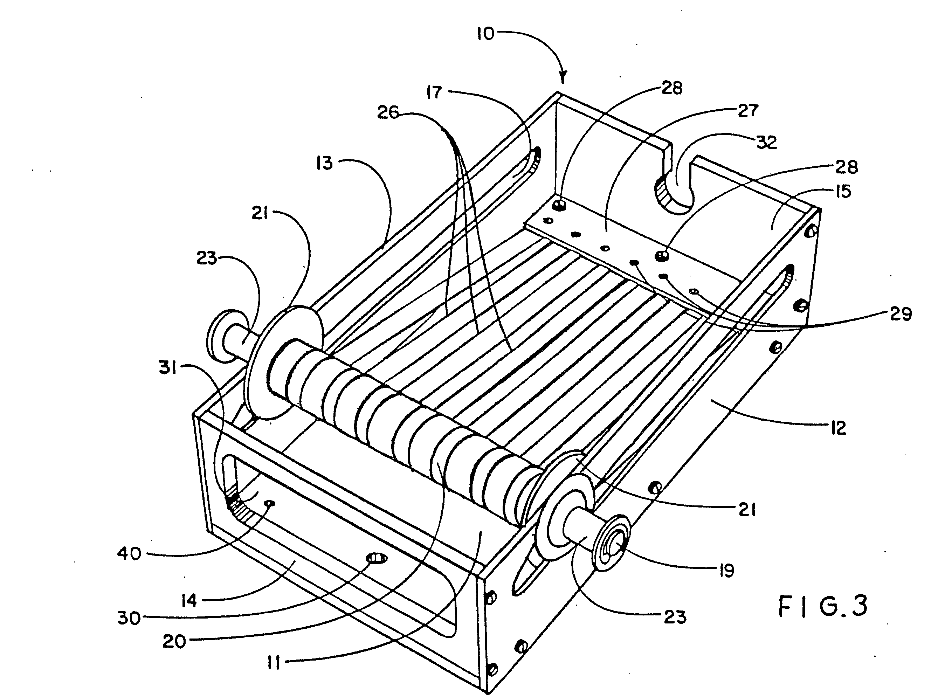

Next, the take up drum 20 is pulled back by manipulating handles 23 to the position shown in Figure 3 which is adjacent angulated offset slots 18. Still manipulating handles 23, the draw bar is moved upwardly into engagement with last mentioned slots. The handles 23 can then be released and the take up drum 20 will remain in relative fixed position due to the constant tension of springs 26.

Next, a conventional infusion bag 34 is laid on springs 26 and the end opposite necks 33 is tucked into the spirals of the coil springs on take up drum 20. The neck 33 of the infusion bag is squeezed and slipped down into the bulbous ended of slot 32. Because of this bulbous portion, such neck will be held in place thereby during use.

Next, the cocking handles 23 are used to disengage the draw bar 19 from the angulated offset slots 18 and to place such draw bar in guide slots 27. The coiling pressure of constant tension springs 26 will cause a predetermined pressure to be developed within the infusion bag.

The infusion needle 38 can then be inserted into the arm 41' of the patient 41 in the normal manner, the proper flow regulating needle 37, having already been selected and inserted as hereinabove described.

The clamp 36 can then be released thereby through the constant pressure created by the constant tension springs 26 and the flow being regulated by the flow regulating needle 37, a predetermined amount of liquid contained within infusion bag 34 can be infused into the patient 41 at a predetermined rate.

To prevent coagulation of blood in the area of the vein orface of the infusion needle 38 when the flow from bag 34 slows or ceases due to the take up drum 20 having been pulled by the constant tension springs 26 to a point adjacent end 15 of the improved infusion means 10 of the present invention, a small rupturable capsule or pouch 43 is provided interiorly of

0248538

infusion bag 34. This capsule 34 will be compressed and ruptured as the take up drum approaches end 15 of the present invention. An anticoagulate such as heparin is contained within the capsule or pouch and once the same has ruptured, will pass through the infusion tube 35, and flow in infusion needles 37 and 38 to prevent blood coagulation for an extended period of time. Once it becomes convenient, or the fact that the infusion bag is empty has been noticed, the same can be replaced as hereinabove described and flow will continue into the patient without having to re-establish communication with the interior of the vein of the patient by the infusion needle 38.

Further, different colored stripes such as that indicated at 44 can be provided on bottom 11 to form a visual alarm system. An example of this would be, as long as only green is visible the infusion bag 34 has plenty of fluid left therein. As it approaches end 15, a yellow warning stripe would become visible, and as it approaches adjacent end 15, a red stripe would also be visible as a warning that the bag needed changing.

In summary, the improved infusion means of the present invention gives a constant predetermined flow rate at all times during dispensing of fluids from the infusion bag 34 as a result of the infusion pump being mounted in a predetermined relationship to the heart of the patient, a predetermined needle orface size which regulates the flow from infusion tube 35, and the constant pressure placed on take up drum 25 by constant tension springs 26.

The present invention may, of course, be carried out in other specific ways than those herein set forth without parting from the spirit and essential characteristics of the invention. The present embodiments are, therefore, to be considered in all respects as illustrative and not restrictive, and all changes coming within the meaning and equivalency range of the appended Claims are intended to be embraced therein.

## CLAIMS

1. Infusion means characterised by: frame means (11 to 15) having guide means (17) associated therewith; take-up drum means (20) operatively associated with said guide means; and at least one coil type substantially constant tension spring means (26) secured at one end to said frame means and unsecuringly wound about said take-up drum at its opposite end, infusion bag means (34), in operation, containing a fluid and being disposable at least partially between the coils of said spring means adjacent said drum means, with infusion tube means (35) communicatively connected or connectible to said infusion bag means.

2. The infusion means claimed in claim 1 in combination with said infusion bag means and tube means, and including fixed orifice, flow regulating means (37) operatively associated with said infusion tube means, whereby, in operation, a substantially constant pressure, predetermined flow rate of fluid from said infusion bag means can be assured.

3. The infusion means claimed in claim 2, wherein said flow regulating means (37) is a flow regulating needle.

4. The infusion means claimed in claim 2 or 3, wherein said flow regulating means (37) is changeable to change the fluid flow rate.

5. The infusion means claimed in any preceding claim, wherein a means (43) for dispensing an anticoagulant into said infusion tube means (35) is provided.

6. The infusion means claimed in claim 5, wherein said means (43) for dispensing said anticoagulant is a rupturable pouch contained within said infusion bag means (34).

7.      The infusion means claimed in claim 6, wherein said pouch is rupturable by said infusion bag means (34) being wound between the coils of said spring means (26) on said take-up drum (20).

8.      A method of infusing a liquid into a patient comprising: placing an infusion bag (34) under or between the coils of substantially constant tension spring means (26) wound on a take-up drum (20); placing an infusion tube (35) in one end of said infusion bag; placing an infusion needle (38) at the end of said infusion tube opposite said infusion bag; inserting said infusion needle into the vein of said patient; and allowing said substantially constant tension spring means to wind up on said infusion bag to create a substantially constant predetermined interior pressure therein.

9.      The method claimed in claim 8, which includes placing a flow regulating orifice (37) of a predetermined size in said infusion tube (35), whereby the rate of fluid flowing from said infusion needle (38) into said patient can be accurately regulated by said orifice.

10.      The method claimed in claim 9, wherein said orifice (37) is in the form of a flow regulating needle.

11.      The method claimed in claim 10, wherein said needle (37) can be changed to change the flow rate of fluid from said infusion bag into said patient.

12.      The method claimed in claim 8, 9, 10 or 11, wherein said infusion bag is mounted at a predetermined location relative to the heart of the patient.

0248538

FIG. 1

FIG. 2

FIG.3

Neu eingereicht / Newly filed
Nouvellement déposé
(R 35)

FIG. 4

FIG. 5

European Patent Office

**PARTIAL EUROPEAN SEARCH REPORT**
which under Rule 45 of the European Patent Convention
shall be considered, for the purposes of subsequent
proceedings, as the European search report

**0248538**

| DOCUMENTS CONSIDERED TO BE RELEVANT | | | EP 87304039.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,X | US - A - 3 647 117 (T.S. HARGEST) | 1 | A 61 M 5/14 |
| Y | * Totality * | 2,3,5-7 | |
| | -- | | |
| Y | US - A - 4 337 769 (R.G. OLSON) | 2,3 | |
| | * Totality; especially fig. 1; column 4, line 67 - column 5, line 4 * | | |
| | -- | | |
| Y | DE - A1 - 3 021 911 (V. LANG) | 5-7 | |
| | * Totality; especially fig. 1b,1c; page 5, last line - page 6, line 8 * | | |
| | -- | | |
| D,X | US - A - 3 670 926 (CH. C. HILL) | 1 | |
| | * Totality * | | TECHNICAL FIELDS SEARCHED (Int. Cl.4) |
| | -- | | |
| P,A | EP - A1 - 0 185 808 (J.R. NAVATO) | 2-4 | A 61 M 5/00 |
| | * Totality; especially fig. 1, ; page 7, lines 31-38 * | | |

**INCOMPLETE SEARCH** --

The Search Division considers that the present European patent application does not comply with the provisions of the European Patent Convention to such an extent that it is not possible to carry out a meaningful search into the state of the art on the basis of some of the claims.

Claims searched completely: 1-7
Claims searched incompletely: -
Claims not searched: 8-12
Reason for the limitation of the search:

In order to Art. 52(4)

| Place of search | Date of completion of the search | Examiner |
|---|---|---|
| VIENNA | 11-09-1987 | LUDWIG |

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | <u>DD - A - 48 434</u> (B. BRAUN)<br><br>  * Totality; especially fig. 1; column 4, lines 23-37 *<br><br>    --- | 2-4 | |
| | | | **TECHNICAL FIELDS SEARCHED (Int. Cl.4)** |